# FASCICULE DE BREVET EUROPEEN

(11) **EP 2 040 779 B1**
(45) Date de publication et mention de la délivrance du brevet: **11.06.2014**
(21) Numéro de dépôt: 07803769.4
(22) Date de dépôt: 26.06.2007
(51) Int. Cl.: A61M 5/20, A61M 5/30, A61M 5/24, A61M 5/31

(54) **DISPOSITIF D'INJECTION SANS AIGUILLE À DOUBLE STOPPEUR À PROFILS DE PRESSION ABAISSÉS**
NADELLOSE INJEKTIONSVORRICHTUNG MIT DOPPELSTOPPER MIT NIEDERDRUCKPROFILEN
NEEDLELESS INJECTION DEVICE WITH DOUBLE STOPPER WITH LOW PRESSURE PROFILES

(30) Priorité: 18.07.2006 FR 0606506
(43) Date de publication de la demande: 01.04.2009
(73) Titulaire: Crossject, 75004 Paris (FR)
(72) Inventeur: ALEXANDRE, Patrick, 70100 Gray (FR)
(74) Mandataire: Gaillarde, Frédéric F. Ch.
(86) Numéro de dépôt international: PCT/FR2007/001055
(87) Numéro de publication internationale: WO 2008/009786

(56) Documents cités:
- WO-A-01/58512
- FR-A1- 2 853 836
- FR-A1- 2 865 407

## Description

Le domaine technique de l'invention est celui des dispositifs d'injection sans aiguille, pré-remplis et jetables, fonctionnant avec une source d'énergie comme, par exemple, un générateur de gaz, et utilisés pour les injections intradermiques, sous-cutanées et intramusculaires, d'un principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection, ou être constitué d'un solide pulvérulent mis en suspension à une certaine concentration dans un liquide approprié. La granulométrie du principe actif doit alors être compatible avec le diamètre des conduits pour éviter de les obturer.

Au sens de la présente demande, l'expression « principe actif liquide » constitue la définition générique couvrant tous les modes de réalisation mentionnés précédemment du principe actif.

Les dispositifs d'injection sans aiguille comportant un réservoir de principe actif liquide en forme de tube existent déjà et ont fait l'objet de brevets. On peut citer, par exemple, la demande de brevet FR 2 853 837 qui se rapporte à un dispositif d'injection sans aiguille comprenant une buse d'injection et un tube destiné à recevoir un principe actif liquide à injecter, ledit tube venant se fixer sur ladite buse à l'aide de moyens de liaison.

On peut citer également la demande de brevet FR 2 853 836 qui se rapporte à une seringue sans aiguille comportant un corps logeant un réservoir cylindrique fermé par un obturateur amont déplaçable et un obturateur aval déplaçable emprisonnant un principe actif et comportant à l'aval un réceptacle avec au moins deux conduits périphériques d'injection, ledit réceptacle en appui sur le réservoir et comprenant un alésage borgne, dont la hauteur libre permet le dégagement des entrées des conduits périphériques lorsque l'obturateur aval est amené au contact du fond de l'alésage dudit réceptacle par le fonctionnement d'un moyen moteur déplaçant l'ensemble obturateur amont-liquide-obturateur aval. Ce dispositif a pour but de résoudre les problèmes d'optimisation des entrées dans les conduits d'injection et de réduire les efforts de pression sur le réceptacle pendant la phase d'injection.

Par ailleurs, la demande de brevet FR 2 865 407 décrit une seringue sans aiguille comportant un corps logeant un réservoir cylindrique fermé par un obturateur amont déplaçable et un obturateur aval déplaçable emprisonnant un principe actif et comportant à l'aval un réceptacle avec au moins un conduit périphérique d'injection, ledit réceptacle en appui sur le réservoir et comprenant un alésage, dans lequel se loge l'obturateur aval quand il est amené au contact du fond de l'alésage dudit réceptacle par le fonctionnement d'un moyen moteur déplaçant l'ensemble obturateur amont-liquide-obturateur aval. Ce type de dispositif permet d'améliorer l'amortissement de l'impact de l'ensemble mobile lorsque l'obturateur aval arrive au contact du fond de l'alésage du réceptacle.

L'homme du métier cherche traditionnellement à minimiser voire éliminer les risques d'endommagement, d'une part, du tube contenant le principe actif liquide, et d'autre part, du stoppeur aval dans la configuration spécifique du «double stoppeur», décrite notamment dans la demande de brevet WO 01/58512. Ladite configuration se caractérise par la présence d'une colonne de liquide délimitée, d'une part, par la paroi latérale du tube et, d'autre part, par un stoppeur amont et un stoppeur aval entre lesquels est logé le principe actif liquide. Sous l'effet de la génération de gaz, ladite colonne se déplace dans le tube jusqu'à ce que le stoppeur aval vienne au contact du fond d'un réceptacle situé dans la buse, de façon à libérer des conduits d'injection pour expulser ledit principe actif. Cependant, lorsque le stoppeur aval impacte le fond dudit réceptacle, il crée une onde de choc qui va se propager jusqu'au tube et dont l'intensité est maximale à l'extrémité dudit tube qui est au contact de la buse. Dans une configuration« double stoppeur », le stoppeur aval et le tube en verre sont les deux éléments les plus sollicités lors du fonctionnement du dispositif d'injection sans aiguille. Ce dernier doit par conséquent être conformé de façon à ce que la vitesse d'impact du stoppeur aval dans le réceptacle soit inférieure à la vitesse limite acceptable pour la tenue mécanique des composants dudit dispositif d'injection. De plus, il est connu que le temps séparant l'ouverture des conduits d'injection et le début de sortie du jet des conduits d'injection doit être plus grand que la durée d'amortissement des oscillations de pression générées par l'ouverture des conduits d'injection.

Il est traditionnellement mentionné dans la littérature du domaine technique en question qu'il est nécessaire d'appliquer au principe actif liquide à injecter, d'une part, une pression élevée comprise entre 200 et 500 bars pour percer la peau, et d'autre part, une pression constante ou décroissante jusqu'en fin d'injection pour assurer le transfert du principe actif liquide, les valeurs finales étant généralement comprises entre 40 et 250 bars.

Cependant, il y a un enjeu majeur à réduire les pressions de fonctionnement afin de construire des dispositifs d'injection sans aiguille résistants et économiques, plus particulièrement pour les dispositifs à usage unique ou les parties consommables, telle que la buse d'injection, des dispositifs réutilisables, tout en garantissant une bonne reproductibilité des injections.

Il a été constaté de façon surprenante que l'objet de la présente invention permet justement d'atteindre d'excellents résultats d'injection sans aiguille avec des profils de pression dont les valeurs initiales sont comprises entre 50 et 150 bars, de préférence entre 70 et 100 bars, c'est-à-dire des valeurs très nettement inférieures à celles communément admises dans le domaine considéré.

Plus précisément, l'objet de la présente invention se rapporte à un dispositif d'injection sans aiguille comprenant une source d'énergie telle qu'un générateur de gaz, un réservoir obturé par un stoppeur amont et un stoppeur aval entre lesquels est logé un principe actif liquide, et une buse d'injection munie d'un réceptacle et d'au moins un conduit d'injection, ledit réceptacle comprenant une cavité dont la hauteur est égale à la distance parcourue par le stoppeur aval avant ouverture de chaque conduit d'injection, caractérisé en ce qu'il est conformé de façon à ce que,
- la hauteur de la cavité en millimètres est comprise entre une hauteur minimale et une hauteur maximale définies respectivement par les relations suivantes,
   ○ hauteur minimale = 3,
   ○ hauteur maximale = 15 x exp. (-(V/9)²) + 10,
   où V est la vitesse de montée initiale du profil de pression exprimée en bars par microseconde,
   le rapport entre la longueur de chaque conduit d'injection et la hauteur de la cavité est compris entre 1 et 2.

Par vitesse de montée initiale en pression, on comprendra qu'il s'agit du calcul dP/dt durant une période de temps allant schématiquement de 0 à 0,5 milliseconde.

De plus, avec un dispositif d'injection sans aiguille selon l'invention, il est possible d'obtenir des profils de pression non obligatoirement plats ou uniformément décroissants, contrairement à ce qui est aujourd'hui communément admis dans la littérature.

Même si plusieurs paramètres comme le nombre et le diamètre des orifices des conduits d'injection ou encore la quantité de principe actif liquide interviennent dans le contrôle de la profondeur de l'injection, un avantage crucial de l'invention réside dans le fait que la grande variété de formes de profil de pression permises en fonction du type de source d'énergie utilisé assure une gestion fine et optimisée de la profondeur de l'injection.

Par ailleurs, un autre avantage considérable d'un dispositif d'injection sans aiguille selon l'invention consiste dans le fait qu'il a été constaté que la pression finale n'a pas d'influence sur la performance dès qu'elle est supérieure à 20 bars, ce qui est à comparer avec les valeurs finales de 40 à 250 bars communément admises dans la littérature.

Grâce aux hauteur de cavité et longueurs d'orifice de sortie déterminées par ces formules mathématiques particulières, il est possible d'établir un compromis entre une valeur de pression initiale que l'on affecte au fluide, ladite pression devant être suffisante pour que le fluide acquière une vitesse de sortie des orifices permettant une pénétration dans les tissus de la peau, et une valeur de pression initiale limitée de façon à ce que l'impact du stoppeur aval sur le réceptacle n'entraîne pas la dégradation des composants mécaniques de la seringue.

Avantageusement, la hauteur minimale de la cavité en millimètres est définie par la relation suivante,
- hauteur minimale = 12 x exp. (-(V/18)²) + 4.

Avantageusement encore, la hauteur maximale de la cavité en millimètres est définie par la relation suivante,
- hauteur maximale = 14 x exp. (-(V/9)²) + 9.

Préférentiellement, le rapport entre la longueur de chaque conduit d'injection et la hauteur de la cavité est compris entre 1,1 et 1,6.

Préférentiellement encore, le dispositif d'injection sans aiguille selon l'invention est conformé de façon à ce que le temps de montée en pression à une valeur de 80 bars est compris schématiquement entre 0,2 et 2,0 millisecondes.

De façon avantageuse, la source d'énergie est constituée par un générateur de gaz pyrotechnique muni d'un chargement pyrotechnique et d'un système d'allumage.

L'invention sera mieux comprise à l'aide de la description détaillée qui est exposée ci-dessous en regard du dessin annexé dans lequel :
La figure 1 est une vue schématique en coupe longitudinale d'un dispositif d'injection sans aiguille selon l'invention ;
Les figures 2 à 5 représentent des exemples de courbes de profils de pression obtenus en fonction de la vélocité de la source d'énergie retenue.

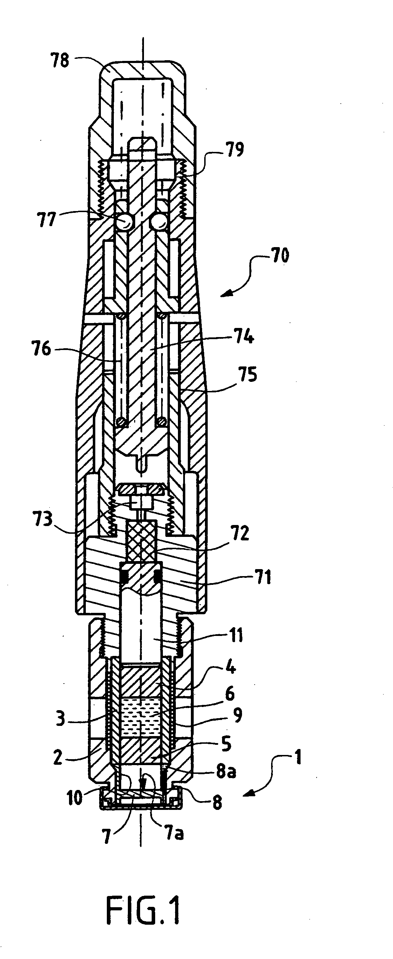

Un dispositif d'injection 1 sans aiguille selon l'invention, tel que représenté à la figure 1, comprend un corps 2 dans lequel est logé un réservoir 3 contenant un principe actif liquide 6.

Une buse d'injection comprenant un réceptacle 7 est disposée à l'extrémité aval du corps 2, le système d'injection étant de façon classique recouvert d'une protection extérieure (non représentée) pour assurer l'asepsie du dispositif d'injection 1.

Un générateur de gaz pyrotechnique 70 contenant un chargement pyrotechnique 72 est fixé par vissage dans l'extrémité amont du corps 2 par l'intermédiaire d'un organe de liaison 71 qui vient en appui contre le réservoir 3, l'étanchéité étant assurée par un joint torique circulaire.

Le corps 2 du dispositif d'injection 1 comporte deux fenêtres diamétralement opposées pour la visualisation du principe actif liquide 6 contenu dans le réservoir 3. A l'aval du corps 2 est emmanché, dans un alésage de forme approprié, le réceptacle 7 qui sera décrit plus en détail par la suite. Le réservoir 3 est en appui sur le réceptacle 7 et centré dans l'aval du corps 2, un matériau intermédiaire 9 transparent étant disposé autour dudit réservoir 3. En amont, le corps 2 reçoit l'organe de liaison 71 qui se centre autour de l'extrémité du réservoir 3. Ce dernier est essentiellement constitué par un tube en verre fermé à ses deux extrémités par un stoppeur amont 4 et un stoppeur aval 5 déplaçables, ceux-ci étant des éléments classiquement utilisés dans des dispositifs d'injection sans aiguille et obtenus par moulage d'élastomères compatibles pour une longue durée avec le principe actif liquide 6, par exemple des chlorobutyl ou bromobutyl dont la dureté Shore est réglée schématiquement entre 45 et 70. Ces éléments peuvent recevoir des traitements de surface notamment pour faciliter leurs déplacements dans le réservoir 3 tubulaire. Au repos, chaque élément a un diamètre supérieur d'environ 10% au diamètre intérieur du réservoir 3, et une hauteur comprise schématiquement entre 0,5 et 0,8 fois ce diamètre. Une fois engagé, chaque élément présente, du fait des déformations subie, une hauteur comprise entre environ 0,6 et 1,0 fois le diamètre intérieur du réservoir 3.

Dans cet exemple de réalisation, le réceptacle 7 est réalisé par un pièce de forme extérieure cylindro-conique qui comporte une cavité 10 centrale dans laquelle va venir se loger le stoppeur aval 5. Sur sa périphérie, le réceptacle 7 comporte trois conduits d'injection 8 uniformément décalés les uns par rapport aux autres. Le diamètre de la cavité 10 centrale est égal à celui du réservoir 3, et sa hauteur libre est égale à celle du stoppeur aval 5. Lorsque ce dernier a atteint le fond 7a du réceptacle 7 du fait de l'actionnement du générateur de gaz pyrotechnique 70, chaque conduit d'injection 8 est alors mis en communication avec le principe actif liquide 6 par le biais d'une entrée 8a, ledit principe actif liquide 6 s'écoulant avec une vitesse correspondant à la pression transmise par le stoppeur amont 4.

Plus précisément, la hauteur de la cavité 10 en millimètres est choisie de façon à être comprise entre une hauteur minimale et une hauteur maximale définies respectivement par les relations suivantes :
- hauteur minimale = 3,
- hauteur maximale = 15 x exp. (-(V/9)²) + 10,
où V est la vitesse de montée initiale du profil de pression exprimée en bars par microseconde. Par vitesse de montée initiale en pression, on comprendra qu'il s'agit du calcul dP/dt durant une période de temps allant schématiquement de 0 à 0,5 milliseconde. De plus, chaque conduit d'injection 8 est conformé de façon à ce que le rapport entre la longueur dudit conduit d'injection 8 et la hauteur de la cavité 10 soit compris entre 1 et 2.

Selon un mode de réalisation préféré, la hauteur de la cavité 10 en millimètres est comprise entre une hauteur minimale et une hauteur maximale définies respectivement par les relations suivantes :
- hauteur minimale = 12 x exp. (-(V/18)²) + 4,
- hauteur maximale = 14 x exp. (-(V/9)²) + 9,
et le rapport entre la longueur de chaque conduit d'injection 8 et la hauteur de la cavité 10 est compris entre 1,1 et 1,6.

Même si plusieurs paramètres comme le nombre et le diamètre des orifices des conduits d'injection 8 ou encore la quantité de principe actif liquide 6 interviennent dans le contrôle de la profondeur de l'injection, il doit être bien compris qu'une grande variété de formes de profils de pression est disponible.

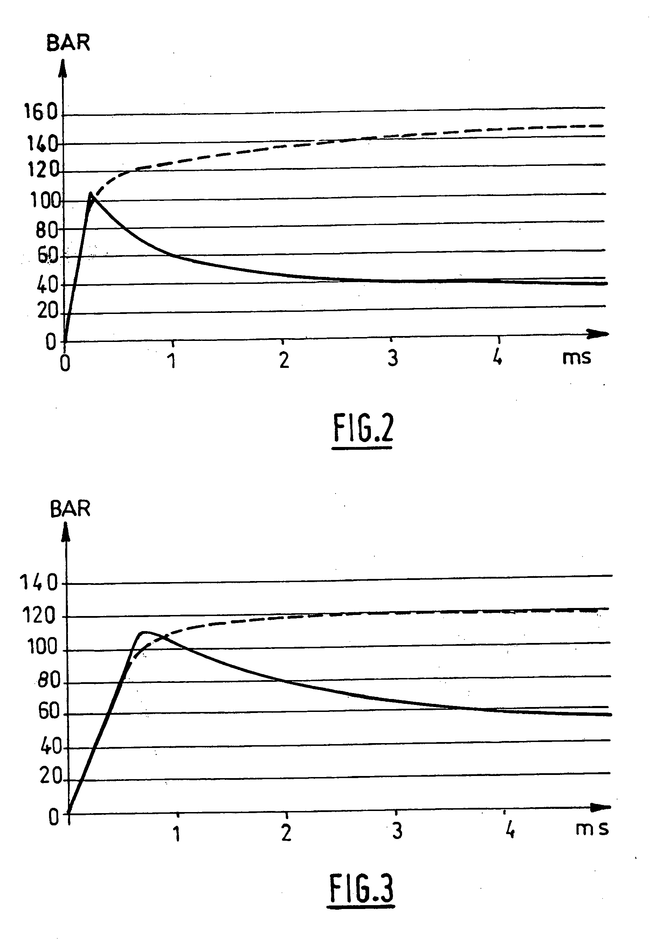

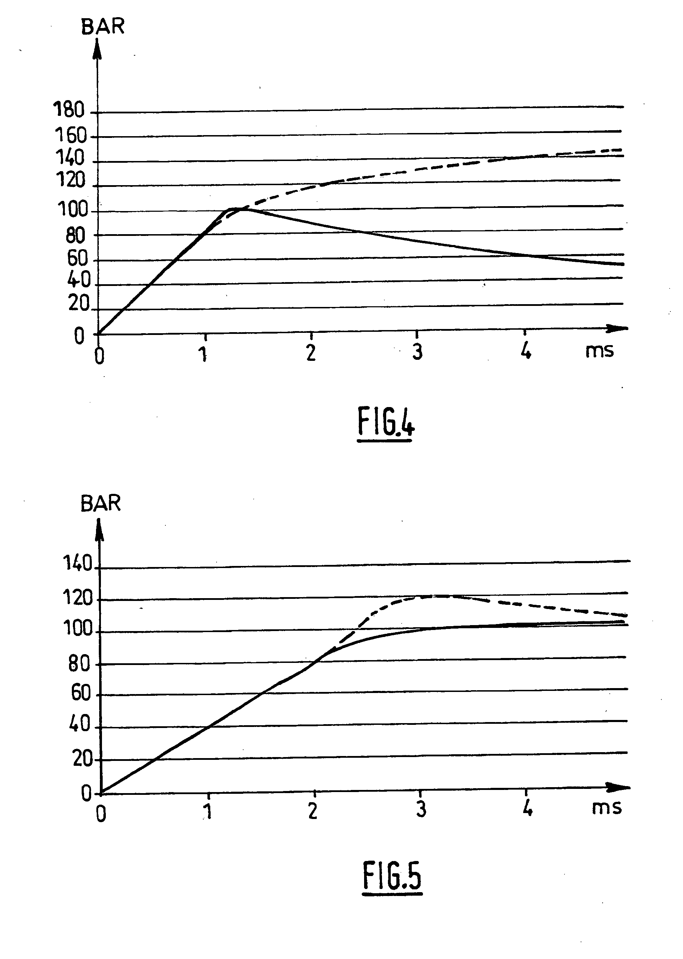

En effet, en se référant aux figures 2 à 5, on s'aperçoit que, aussi bien pour un chargement pyrotechnique 72 à forte vivacité, dont le profil de pression est représenté à la figure 2, et qui est caractérisé par un temps de montée en pression à une valeur de 80 bars de l'ordre de 0,2 millisecondes, que pour d'autres chargements pyrotechniques 72 à moyenne, faible et très faible vivacités, dont les profils de pression sont représentés respectivement aux figures 3 à 5, et qui sont caractérisés par des temps de montés en pression à une valeur de 80 bars de l'ordre respectivement de 0,5 milliseconde, 1,0 milliseconde et 2,0 millisecondes, il est possible d'obtenir des profils de pression différents pour une vivacité donnée selon le type d'amorce 73 choisi pour initier le chargement pyrotechnique 72. A titre d'illustration, dans chacune des configurations représentées aux figures 2 à 5, deux exemples de courbes de profils de pression différents sont tracés. La courbe du profil de pression préférentiel dont la pression finale est la plus faible est représentée en trait continu, et la courbe du profil de pression dont la pression finale est la plus forte est représentée en pointillés.

Il a été constaté que l'on peut par exemple réaliser des injections sous-cutanées tout à fait satisfaisantes de 0,5 millilitre de principe actif liquide 6 avec une pression de l'ordre de 80 bars en début d'injection et de 30 bars en fin d'injection, pour un dispositif d'injection 1 muni de trois conduits d'injection 8 de 250 micromètres de diamètre. Bien évidemment, les valeurs de pression et le nombre/diamètre des conduits d'injection 8 sont adaptés à la quantité de principe actif liquide 6 à injecter ainsi qu'à sa viscosité, et à la profondeur d'injection souhaitée.

Dans cette réalisation, le générateur de gaz pyrotechnique 70 agit sur la stoppeur amont 4 par l'intermédiaire d'un piston 11 de section efficace égale à celle dudit stoppeur amont 4. Ce piston 11 étant en contact avec le stoppeur amont 4, il n'y a donc pas d'effet de choc ou de bélier en début de fonctionnement. Ce piston 11, grâce à son système d'étanchéité, empêche les gaz générés par la combustion du chargement pyrotechnique 72 de venir en contact avec le stoppeur amont 4, et donc permet d'éviter d'éventuelles détériorations de celui-ci ainsi que des fuites de gaz vers le principe actif liqude 6 contenu dans le réservoir 3. Le piston 11, d'une couleur adaptée, peut aussi servir d'indicateur de fonctionnement en apparaissant dans les fenêtres de visualisation du corps 2.

Nous allons décrire à ce stade les principaux éléments du générateur de gaz pyrotechnique 70, en sus du chargement pyrotechnique 72 et de l'amorce 73. Plus précisément, le générateur de gaz pyrotechnique 70 comprend un organe de liaison 71 disposé autour du piston 11, et dans lequel est placé le chargement pyrotechnique 72, juste au-dessus dudit piston 11. L'amorce 73 surmonte le chargement pyrotechnique 72 dont la combustion est initiée lorsque ladite amorce 73 est impactée par un percuteur 74. En position initiale, le percuteur 74 est retenu dans un guide-percuteur 75 vissé dans l'organe de liaison 71 par des billes 77 partiellement engagées dans une gorge du percuteur 74. Un dispositif de percussion est également prévu et se décompose en un poussoir 78 présentant une gorge élargie 79, et un ressort 76 intérieur. Le poussoir 78 coulisse sur l'extérieur du guide-percuteur 75, et il est retenu par des ergots se déplaçant dans des rainures latérales. Ce poussoir 78 constitue dans cet exemple l'organe de déclenchement.

Bien évidemment, pour initier la combustion du chargement pyrotechnique 72, sans sortir du cadre de l'invention, il est possible d'utiliser des dispositifs d'initiation autres que le dispositif à percuteur décrit précédemment. Sans entrer dans les détails et sans chercher à être exhaustif, nous citerons comme exemples des dispositifs d'initiation à pile électrique ou des dispositifs d'initiation piezoélectrique.

Eventuellement, le générateur de gaz pyrotechnique 70 peut être remplacé par un générateur de gaz constitué par un réservoir de gaz comprimé fermé par une vanne à ouverture rapide. L'organe de déclenchement viendra ouvrir ladite vanne, et les gaz comprimés du réservoir pourront alors de détendre et agir sur la moyen de poussée.

Pour l'utilisation du dispositif d'injection 1 selon l'invention, après avoir enlevé le bouchon d'asepsie, et posé la face aval dudit dispositif d'injection 1 sur la peau du sujet à traiter, l'opérateur appuie, avec son pouce, sur le poussoir 78 qui s'enfonce en comprimant le ressort 76. Le poussoir 78 se déplace en translation jusqu'à ce que la gorge élargie 79 du percuteur 74 arrive à la hauteur des billes 77. Ce faisant, ces dernières s'engagent sous l'effet de la pesanteur dans la gorge élargie 79 et libèrent de ce fait le percuteur 74 qui va alors impacter violemment l'amorce 73 dont l'initiation enflamme le chargement pyrotechnique 72. Les gaz générés par ce dernier vont de façon connue forcer la colonne constituée par le stoppeur amont 4, le principe actif liquide 6, et le stoppeur aval 5 à coulisser le long du réservoir 3 jusqu'à ce que le stoppeur aval 5 vienne en butée contre le fond 7a du réceptacle 7. Sous l'effet de la déformation, le stoppeur aval 5 libère les entrées 8a des conduits d'injection 8, et permet donc au principe actif liquide 6 d'être injecté à grande vitesse afin de pouvoir traverser la peau de la personne à traiter.

Bien que l'invention ait été décrite en liaison avec des exemples particuliers de réalisation, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Dispositif d'injection (1) sans aiguille comprenant une source d'énergie telle qu'un générateur de gaz, un réservoir (3) obturé par un stoppeur amont (4) et un stoppeur aval (5) entre lesquels est logé un principe actif liquide (6), et une buse d'injection munie d'un réceptacle (7) et d'au moins un conduit d'injection (8), ledit réceptacle comprenant une cavité (10) dont la hauteur est égale à la distance parcourue par le stoppeur aval avant ouverture de chaque conduit d'injection, le rapport entre la longueur de chaque conduit d'injection et la hauteur de la cavité est compris entre 1 et 2 **caractérisé en ce qu'**il est conformé de façon à ce que,
- la hauteur de la cavité en millimètres est comprise entre une hauteur minimale et une hauteur maximale définies respectivement par les relations suivantes,
• hauteur minimale = 3,
• hauteur maximale = 15 x exp. (-(V/9)²) + 10,
où V est la vitesse de montée initiale du profil de pression exprimée en bars par microseconde.

2. Dispositif d'injection (1) selon la revendication 1, **caractérisé en ce que** la hauteur minimale de la cavité (10) en millimètres est définie par la relation suivante,
• hauteur minimale = 12 x exp. (-(V/18)²) + 4.

3. Dispositif d'injection (1) selon l'une quelconque des revendications 1 ou 2, **caractérisé en ce que** la hauteur maximale de la cavité (10) en millimètres est définie par la relation suivante,
• hauteur maximale = 14 x exp. (-(V/9)²) + 9.

4. Dispositif d'injection (1) selon l'une quelconque des revendications 1 à 3, **caractérisé en ce que** le rapport entre la longueur de chaque conduit d'injection (8) et la hauteur de la cavité (10) est compris entre 1,1 et 1,6.

5. Dispositif d'injection (1) selon l'une quelconque des revendications 1 à 4, **caractérisé en ce qu'**il est conformé de façon à ce que le temps de montée en pression à une valeur de 80 bars est compris entre 0,2 et 2,0 millisecondes.

6. Dispositif d'injection (1) selon l'une quelconque des revendications 1 à 5, **caractérisé en ce que** la source d'énergie est constituée par un générateur de gaz pyrotechnique (70) muni d'un chargement pyrotechnique (72) et d'un système d'allumage.

## Patentansprüche

1. Verfahren zur Injektion (1) ohne Nadel, umfassend eine Energiequelle wie z.B. einen Gasgenerator, einen Behälter (3), der durch eine vorgelagerte Stoppvorrichtung (4) und eine nachgelagerte Stoppvorrichtung (5) verschlossen ist, zwischen denen ein flüssiger Wirkstoff (6) gelagert ist, und eine Injektionsdüse, die mit einem Auffangbecken (7) und mindestens einer Injektionsleitung (8) ausgestattet ist, wobei das Auffangbecken einen Hohlraum (10) umfasst, dessen Höhe gleich dem Abstand ist, der von der nachgelagerten Stoppvorrichtung vor der Öffnung jeder Injektionsleitung zurückgelegt wird, wobei das Verhältnis zwischen der Länge jeder Injektionsleitung und der Höhe des Hohlraums zwischen 1 und 2 liegt, **dadurch gekennzeichnet, dass** es derart eingestellt ist, dass
- die Höhe des Hohlraums in Millimetern zwischen einer minimalen Höhe und einer maximalen Höhe liegt, jeweils definiert durch die folgenden Verhältnisse:
- minimale Höhe = 3,
- maximale Höhe = 15 x exp. (-(V/9)²) + 10,
wobei V die Geschwindigkeit des anfänglichen Anstiegs des Druckprofils ist, ausgedrückt in Bar pro Millisekunde.

2. Injektionsvorrichtung (1) nach Anspruch 1, **dadurch gekennzeichnet, dass** die minimale Höhe des Hohlraums (10) in Millimetern durch das folgende Verhältnis definiert wird:
- minimale Höhe = 12 x exp. (-(V/18)²) + 4.

3. Injektionsvorrichtung (1) nach einem der Ansprüche 1 oder 2, **dadurch gekennzeichnet, dass** die maximale Höhe des Hohlraums (10) in Millimetern durch die folgende Beziehung definiert wird:
- maximale Höhe = 14 x exp. (-(V/9)²) + 9.

4. Injektionsvorrichtung (1) nach einem der Ansprüche 1 bis 3, **dadurch gekennzeichnet, dass** das Verhältnis zwischen der Länge jeder Injektionsleitung (8) und der Höhe des Hohlraums (10) zwischen 1,1 und 1,6 liegt.

5. Injektionsvorrichtung (1) nach einem der Ansprüche 1 bis 4, **dadurch gekennzeichnet, dass** sie derart eingestellt ist, dass die Zeit des Druckanstiegs auf einen Wert von 80 Bar zwischen 0,2 und 2,0 Millisekunden liegt.

6. Injektionsvorrichtung (1) nach einem der Ansprüche 1 bis 5, **dadurch gekennzeichnet, dass** die Energiequelle aus einem pyrotechnischen Gasgenerator (70) besteht, der mit einer pyrotechnischen Ladung (72) und einem Zündsystem ausgestattet ist.

## Claims

1. A needle-free injection device (1) comprising an energy source such as a gas generator, a reservoir (3) closed by an upstream stopper (4) and a downstream stopper (5) between which is accommodated a liquid active principle (6), and an injection nozzle provided with a receptacle (7) and with at least one injection conduit (8), said receptacle (7) comprising a cavity (10), the height of which is equal to the distance travelled by the downstream stopper prior to the opening of each injection conduit, the ratio between the length of each injection conduit and the height of the cavity is comprised between 1 and 2, **characterized in that** it is shaped so that,
- the height of the cavity in millimeters is comprised between a minimum height and a maximum height defined respectively by the following relationships:
• minimum height = 3,
• maximum height = 15 x exp. (-(V/9)²) + 10,
where V is the initial rate of rise of the pressure profile expressed in bar per microsecond.

2. The injection device (1) according to claim 1, **characterized in that** the minimum height of the cavity (10) in millimeters is defined by the following relationship:
• minimum height = 12 x exp. (-(V/18)²) + 4

3. The injection device (1) according to any one of claim 1 or 2, **characterized in that** the maximum height of the cavity (10) in millimeters is defined by the following relationship:
• maximum height = 14 x exp. (-(V/9)²) + 9

4. The injection device (1) according to any one of claims 1 to 3, **characterized in that** the ratio between the length of each injection conduit (8) and the height of the cavity (10) is comprised between 1.1 and 1.6.

5. The injection device (1) according to any one of claims 1 to 4, **characterized in that** it is shaped so that the pressure rise time to a value of 80 bars is comprised between 0.2 and 2.0 milliseconds.

6. The injection device (1) according to any one of claims 1 to 5, **characterized in that** the energy source is composed of a pyrotechnic gas generator (70) provided with a pyrotechnic charge (72) and an ignition system.
